**Europäisches Patentamt**

**European Patent Office**

**Office européen des brevets**

(19)

(11) Veröffentlichungsnummer : **0 149 234**
**B1**

(12) # EUROPÄISCHE PATENTSCHRIFT

(45) Veröffentlichungstag der Patentschrift :
**15.06.88**

(51) Int. Cl.⁴ : **B 62 B   3/00, A 61 L   2/26**

(21) Anmeldenummer : **84116278.7**

(22) Anmeldetag : **24.12.84**

(54) Verfahren und Vorrichtung zum Transport von Sterilisationseinheiten für Operationswäsche.

(30) Priorität : **14.01.84 DE 3401109**

(43) Veröffentlichungstag der Anmeldung :
**24.07.85 Patentblatt 85/30**

(45) Bekanntmachung des Hinweises auf die Patenterteilung : **15.06.88 Patentblatt 88/24**

(84) Benannte Vertragsstaaten :
**AT BE CH DE FR GB IT LI NL SE**

(56) Entgegenhaltungen :
**DE-B- 1 271 563**
**DE-B- 2 543 771**
**FR-A- 2 325 548**
**US-A- 3 698 735**
**US-A- 4 073 663**

(73) Patentinhaber : **adrett-Reinigung GmbH**
**Herzforder Strasse 9**
**D-4450 Lingen 1 (DE)**

(72) Erfinder : **Göhman, Uwe, Dipl.-Ing.**
**Baumblütenweg 1**
**D-4450 Lingen (DE)**

(74) Vertreter : **Habbel, Hans-Georg, Dipl.-Ing.**
**Postfach 3429 Am Kanonengraben 11**
**D-4400 Münster (DE)**

EP 0 149 234 B1

## Beschreibung

Die Erfindung bezieht sich auf ein Verfahren zum sterilen Transport von Sterilisationseinheiten und eine Vorrichtung zur Durchführung dieses Verfahrens.

Bei der Sterilisation von Operationswäsche tritt das Problem auf, daß die Sterilisation der Wäsche in einem vom Operationsraum entfernten Sterilisator erfolgt. Hierbei wird die Wäsche in sogenannten Sterilisationseinheiten untergebracht, d. h. entsprechend ausgebildeten, genormten Behältern, die die Sterilisation der Wäsche im Sterilisator ermöglichen und den Transport gewährleisten, ohne daß dabei eine Kontaminierung des Behälterinnenraumes erfolgt.

Die derart sterilisierten Behälter werden heute auf mit Lauf- bzw. Lenkrollen ausgerüstete Transportwagen, z. B. gemäß der FR-A-24 97 095 und der FR-A-25 21 425, mit Traggestellen bzw. Führungsschienen für die Sterilisationseinheiten geladen und dann ohne jede zusätzliche Schutzmaßnahme vom Sterilisator zum Operationsraum verfahren. Hierbei hat sich herausgestellt, daß auf diesem Weg eine erhebliche Kontaminierung der Außenseite der Sterilisationsbehälter oder -einheiten erfolgt, die dazu führen kann, daß beim Öffnen der Sterilisationseinheiten im Operationsraum auch eine Kontaminierung des Behälterinnenraumes und des Behälterinhaltes erfolgen kann.

Der Erfindung liegt die Aufgabe zugrunde, ein Verfahren und eine Vorrichtung zu schaffen, mit der es möglich ist, die im Sterilisator sterilisierten Sterilisationseinheiten ohne die Gefahr der Kontaminierung der Behälteraußenseite vom Sterilisator zum Operationsraum zu verbringen.

Diese der Erfindung zugrundeliegende Aufgabe wird durch die Verfahrensschritte gemäß dem Anspruch 1 und durch eine Vorrichtung gemäß dem Anspruch 2 gelöst.

Mit anderen Worten ausgedrückt : Die Sterilisationseinheiten werden auf einen Trägerwagen gestapelt, der Trägerwagen seinerseits wieder auf einen Transportwagen gestellt und beide Wagen werden mit je einer eigenen textilen Schutzumhüllung versehen. Hierbei ist es bereits aus der US-A-40 77 535 bekannt, im Krankenhaus Transportvorrichtungen vorzusehen, die aus einem Trägerwagen mit darauf befindlichen Transportwagen bestehen.

Die derart ineinander gestapelten Sterilisationseinheiten, Trägerwagen und Transportwagen werden im Sterilisator sterilisiert und dann in dieser sterilisierten Form vom Sterilisator zum Operationsraum verfahren. Der Transportwagen wird dann vor der Schleuse des Operationsraumes abgestellt und die den Transportwagen umgebende Textilumhüllung abgenommen. Aus der Schleuse heraus wird dann der noch mit seiner Textilumhüllung versehene Trägerwagen über eine Auffahrrampe oder sonstige Hilfsmittel vom Transportwagen in den Operationsraum verfahren. Hier wird die textile Umhüllung des Trägerwagens abgenommen, die nunmehr nicht mehr mit der Umgebungsluft in Kontakt gekommen ist und nur von einer Person gehandhabt wurde, die sich innerhalb des Operationsraumes befand, also bereits sterile Kleidung trug und entsprechende Vorsichtsmaßnahmen, wie Handschuhe od. dgl. angewendet hat. Nachdem die textile Umhüllung des Trägerwagens entfernt ist, sind nunmehr die einzelnen, auf dem Trägerwagen gestapelten Sterilisationseinheiten zugänglich und können entleert werden.

Hieraus wird ersichtlich, daß jede Kontaminierung der eigentlichen Sterilisationseinheiten an deren Außenseite nicht mehr möglich ist, weil die Sterilisationseinheiten auf dem Transport vom Sterilisator bis zum Operationsraum keiner Kontaminierung mehr unterliegen konnten.

Vorteilhafte Ausgestaltungen der Erfindung sind in den Unteransprüchen erläutert.

Ein Ausführungsbeispiel der Erfindung wird nachfolgend anhand der Zeichnungen beschrieben. Die Zeichnungen zeigen dabei in

Fig. 1 schaubildlich einen Transportwagen, einen Trägerwagen und eine Sterilisationseinheit, in

Fig. 2 schaubildlich den Transportwagen und eine Auffahrrampe, in

Fig. 3 einen Transportwagen mit aufgesetztem Trägerwagen und über den Trägerwagen gesetzte haubenartige Umhüllung, in

Fig. 4 in größerem Maßstab die Riegelmittel zur Verriegelung der Auffahrrampe am Transportwagen und zur Verriegelung des Trägerwagens auf dem Transportwagen in einer Stellung, in der die Auffahrrampe verriegelt und der Trägerwagen freigegeben ist, und in

Fig. 5 eine Ansicht auf die Einrichtung gemäß Fig. 4, bei welcher der Trägerwagen verriegelt ist, aber die Auffahrrampe freigegeben ist.

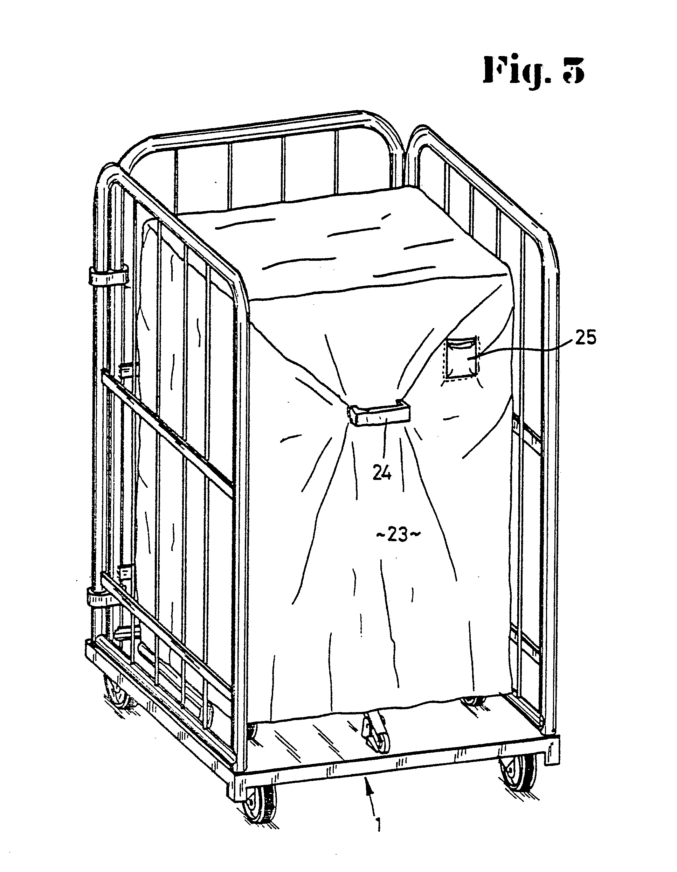

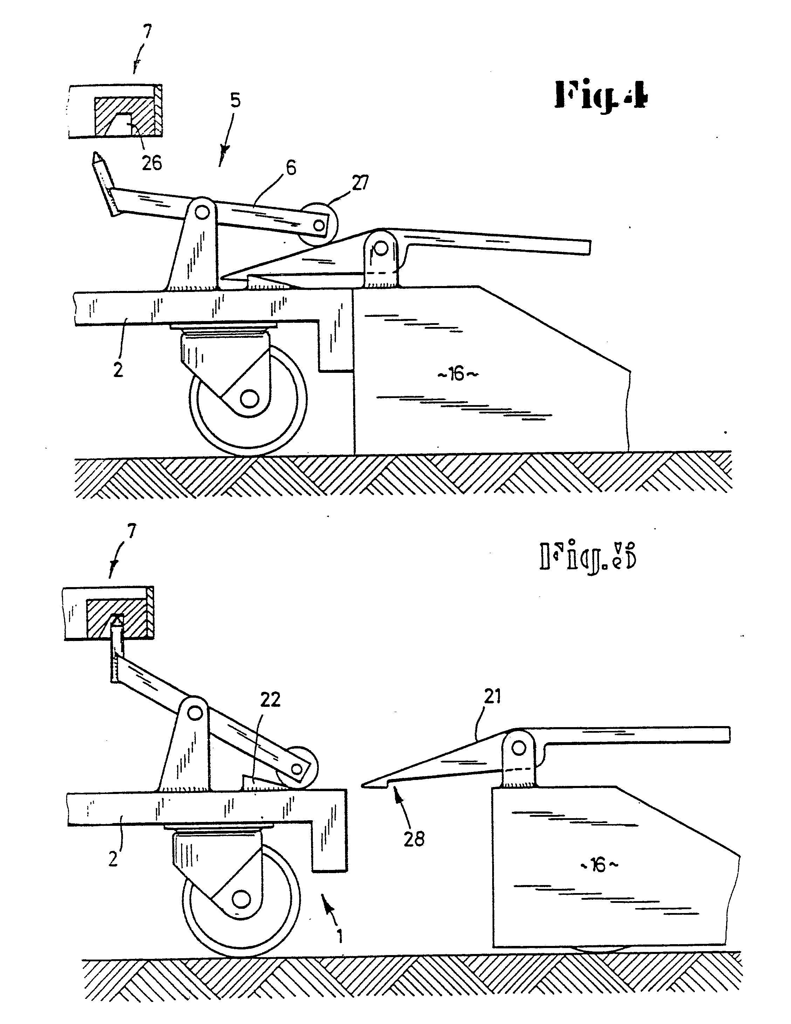

In den Zeichnungen ist mit 1 ein Transportwagen bezeichnet, der im wesentlichen aus einer Grundplatte 2 besteht, die mit Laufrollen 3 ausgerüstet ist. Nach oben hin schließen sich an die Grundplatte 2 Gitterelemente 4 an, die den Transportwagen dreiseitig umgeben und damit an einer Seite offen lassen. Auf der Grundplatte 2 ist eine Arretierung 5 vorgesehen, die aus einem Kipphebel 6 besteht, der nachfolgend anhand den Fig. 4 und 5 noch zu erläutern sein wird.

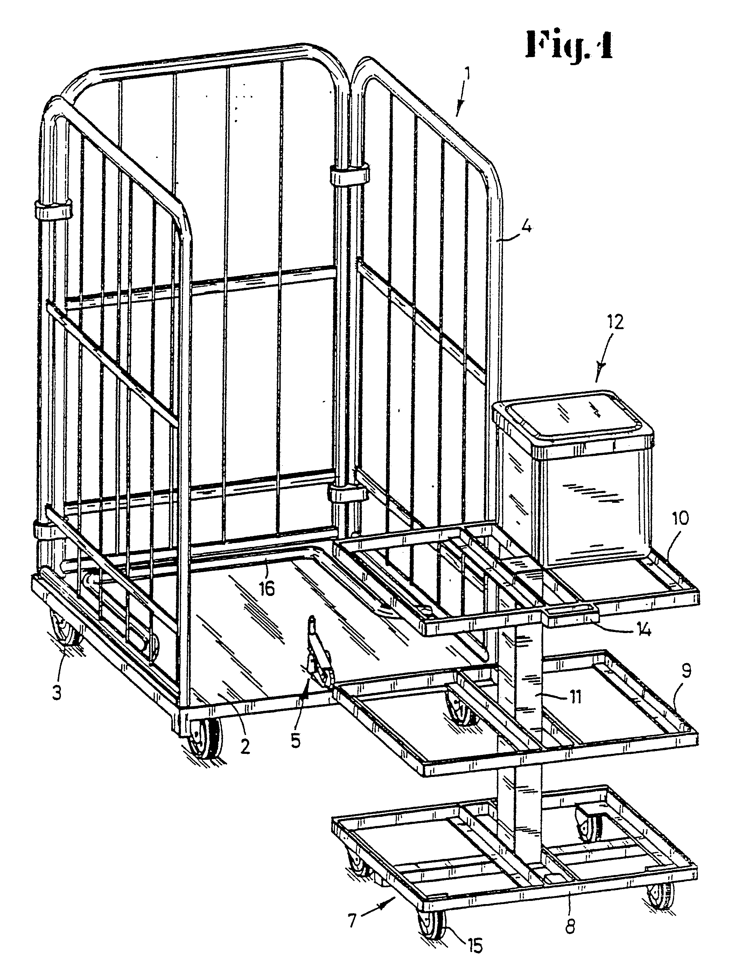

Ein Trägerwagen 7 besteht aus rahmenartigen Traggestellen 8, 9 und 10, die an einer zentralen Tragsäule 11 angeordnet sind. Die Traggestelle 8, 9 und 10 sind dabei matrizenförmig ausgebildet und nehmen Sterilisationseinheiten auf, von denen eine in Fig. 1 dargestellt und mit 12 bezeichnet ist. Die Sterilisationseinheiten können in ihrer Größe auch an die Größe der rahmenartigen Traggestelle 8, 9 und 10 angepaßt sein.

An dem oberen rahmenartigen Traggestell 10 ist ein über die vordere Horizontalflucht des Trägerwagens 7 vorstehender Griff 14 angeordnet, mit dem der Trägerwagen 7 betätigt werden kann.

Der Trägerwagen 7 weist ebenfalls Laufrollen 15 auf, von denen wenigstens eine oder mehrere als Lenkrollen ausgebildet sein können, ebenso wie dies bei den Laufrollen 3 für den Transportwagen der Fall ist.

Auf der Grundplatte 2 des Transportwagens ist eine schienenartige Führung 16 vorgesehen, die der genauen Zentrierung des Trägerwagens 7 auf der Grundplatte 2 des Transportwagens dient.

Im aufgesetzten Zustand des Trägerwagens 7 auf dem Transportwagen 1 kann der Transportwagen durch die Arretierung 5 auf dem Transportwagen festgelegt werden.

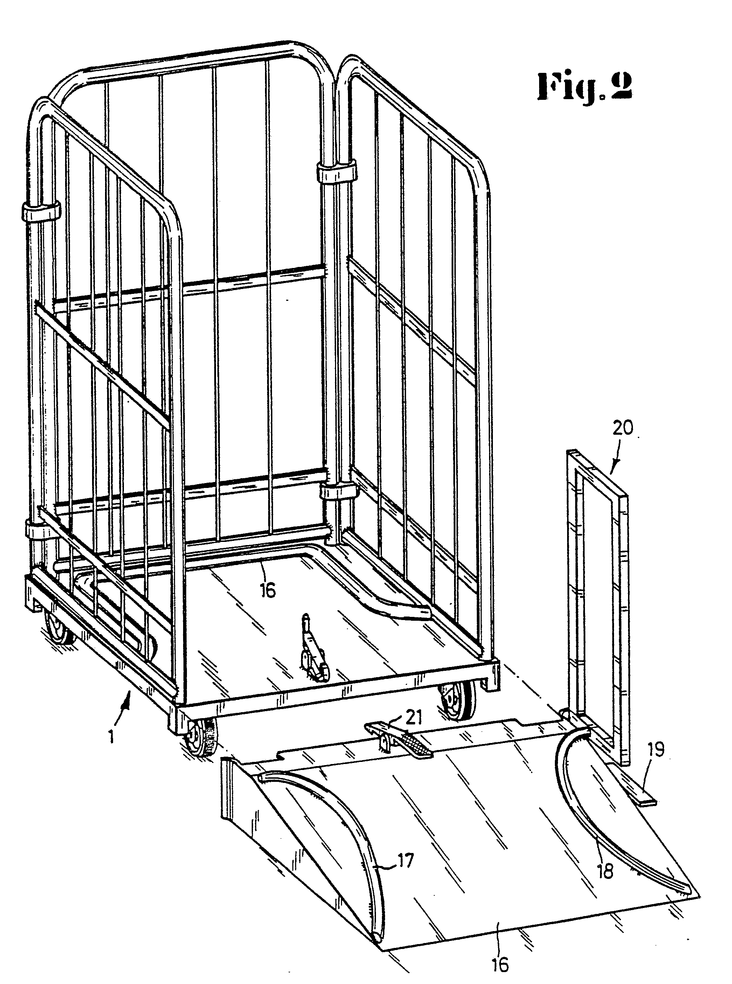

In Fig. 2 ist eine Auffahrrampe 16 dargestellt, die ebenfalls der Führung des Trägerwagens 7 dienende Führungsschienen 17 und 18 aufweist. Die Auffahrrampe 16 ist mit aus der Zeichnung nicht erkennbaren Laufrollen versehen, die über einen beispielsweise fußbetätigten Stellhebel 19 arretiert werden können. Die Auffahrrampe 16 kann über den Handbetätigungsgriff 20 betätigt, d. h. verfahren werden.

An der Auffahrrampe 16 ist ein Riegel 21 vorgesehen, mit dem die Auffahrrampe 16 an dem Transportwagen 1 festgelegt werden kann, wobei dieser Riegel 21 hinter einen entsprechenden Riegelnocken 22 auf der Grundplatte 2 des Transportwagens 1 greift (Fig. 5).

In Fig. 3 ist eine haubenartige Umhüllung 23 dargestellt, die in ihrer Größe der Umfangsgröße des Trägerwagens angepaßt ist und eine Ausformung 24 für den Griff 14 des Trägerwagens aufweist. Außerdem ist diese haubenartige Umhüllung 23 mit einer Tasche 25 ausgerüstet, die der Aufnahme von Transportpapieren od. dgl. dient.

In der Zeichnung ist die haubenartige Umhüllung für den Transportwagen aus Übersichtlichkeitsgründen nicht dargestellt, sie ist aber ein wesentliches Bestandteil des erfindungsgemäßen Systems.

In Fig. 4 ist ein Teil des Trägerwagens 7 erkennbar. Die auf dem Transportwagen angebrachte Arretierung greift mit dem Kipphebel 6 in eine entsprechende Aufnahmeöse 26 des Trägerwagens 7 ein, dann, wenn der Kipphebel 6 in der in Fig. 5 dargestellten Stellung steht. Wird nunmehr die Auffahrrampe 16 an den Transportwagen 1 herangeschoben, greift der Riegel 21 unter die Laufrolle 27 des Kipphebels 6, hebt diesen an und führt ihn aus der Verriegelung mit dem Tragerwagen 7. Gleichzeitig greift eine Riegelnase 28 des Riegelhebels 21 hinter den Riegelnocken 22 und verriegelt dadurch die Auffahrrampe 16 mit der Grundplatte 2 des Transportwagens 1.

Die Arbeitsweise mit dem vorbeschriebenen Bausatz ist wie folgt :

Die zu sterilisierende Wäsche wird in die Sterilisationseinheiten 12 eingefüllt, diese werden verschlossen und dann auf den Trägerwagen 7 aufgesetzt. Anschließend wird der Trägerwagen 7 mit der haubenartigen Umhüllung 23 versehen und auf den Transportwagen 1 aufgesetzt. Anschließend wird auch der Transportwagen 1 mit einer in der Zeichnung nicht dargestellten haubenartigen Umhüllung ausgerüstet. In dieser Form gelangt der Transportwagen mit dem aufgesetzten Trägerwagen 7 in den Sterilisator.

Anschließend wird der immer noch mit der haubenartigen Umhüllung versehene Transportwagen 1 aus dem Sterilisator zum Operationsraum verfahren. Im Bereich der Schleuse des Operationsraumes befindet sich die Auffahrrampe 16, die dann an den Transportwagen 1 angeschoben und mit diesem verriegelt wird. Hierbei löst sich gleichzeitig der Kipphebel 6 aus dem Trägerwagen 7, so daß nach Abnahme der haubenartigen Umhüllung vom Transportwagen 1 nunmehr der immer noch mit einer haubenartigen Umhüllung 23 versehene Trägerwagen 7 über die Auffahrrampe 16 aus dem Transportwagen 1 herausgezogen und in den Operationsraum verfahren werden kann. Hierbei erfolgt die Betätigung des Trägerwagens 7 aus dem Bereich des Operationsraumes heraus, indem der Trägerwagen 7 an dem Bereich 24, d. h. seinem Griff 14, ergriffen wird.

Erst im Operationsraum wird dann die haubenartige Umhüllung 23 vom Trägerwagen 7 abgenommen und nunmehr können innerhalb des Operationsraumes die Sterilisationseinheiten 12 transportiert werden.

## Patentansprüche

1. Verfahren zum Transport von in Operationsräumen einzusetzenden sterilen Wäscheteilen unter Verwendung von sogegenannten Sterilisationseinheiten, die als Behälter für die Wäsche ausgebildet sind und die in einem vom Operationsraum entfernten Sterilisator behandelt worden sind, dadurch gekennzeichnet, daß die Sterilisationseinheiten in einem Trägerwagen gestapelt werden, der Trägerwagen mit einer haubenartigen Textilumhüllung überzogen wird, der derart ausgebildete Trägerwagen auf einen Transportwagen aufgesetzt, der Transportwagen mit einer haubenartigen Textilumhüllung versehen wird und die so ineinander geschachtelten Wagen im Sterilisator behandelt und anschließend zum Operationsraum verfahren werden, wobei nach Abnahme der haubenartigen Textilumhüllung des Transportwagens der mit der haubenartigen Textilumhüllung versehene Trägerwagen mit den darauf angeordneten Sterilisationseinheiten in den Operationsraum transportiert wird.

2. Vorrichtung zum sterilen Transport von Sterilisationseinheiten für Operationswäsche od. dgl. vom Sterilisator zum Operationsraum gemäß dem Verfahren nach Anspruch 1, gekennzeichnet durch

a) einen Trägerwagen (7) mit Halterungen für die Sterilisationseinheiten (12),

b) einen Transportwagen (1) zur Aufnahme des Trägerwagens (7) mit Arretierungen (5) für den auf dem Transportwagen (1) angeordneten Trägerwagen (7),

c) je eine aus textilem Werkstoff bestehende haubenartige Umhüllung (23) für den Trägerwagen (7) und den Transportwagen (1).

3. Vorrichtung nach Anspruch 2, dadurch gekennzeichnet, daß der Trägerwagen (7) aus einem oder mehreren horizontalen und rahmenartigen Traggestellen (8, 9, 10) für die Sterilisationseinheiten (12) gebildet ist, wobei die Traggestelle (8, 9, 10) an einer zentralen Tragsäule (11) angeordnet sind und das unterste Traggestell (8) mit Laufrollen (15) versehen ist.

4. Vorrichtung nach Anspruch 2 und 3, dadurch gekennzeichnet, daß eine oder mehrere der Laufrollen (3, 15) als Lenkrollen ausgebildet sind.

5. Vorrichtung wenigstens nach Anspruch 3, dadurch gekennzeichnet, daß an einem der rahmenartigen Traggestelle (10) ein über die vordere Horizontalflucht des Trägerwagens (7) vorstehender Griff (14) angeordnet ist.

6. Vorrichtung nach Anspruch 2, dadurch gekennzeichnet, daß der Transportwagen (1) aus einer mit Laufrollen (3) versehenen Grundplatte (2) und einem darauf nach oben anschließenden, den Transportwagen (1) dreiseitig umschließenden Gittergestell (4) aufgebaut ist.

7. Vorrichtung nach Anspruch 2 und 3, gekennzeichnet durch eine Auffahrrampe (16) zur Verbindung der Grundplatte (2) des Transportwagens (1) mit einem Gebäudeboden (Fig. 2).

8. Vorrichtung nach Anspruch 7, gekennzeichnet durch Führungsschienen (17, 18) zur Führung der Rollen (15) des Trägerwagens (7) auf der Auffahrrampe (16) (Fig. 2).

9. Vorrichtung nach einem der vorhergehenden Ansprüche 2-8, gekennzeichnet durch an dem Trägerwagen (7) und dem Transportwagen (1) angeordnete, miteinander zusammenwirkende Riegel (6, 26), die bei auf dem Transportwagen (1) aufgesetzten Trägerwagen (7) diesen in seiner auf dem Transportwagen (1) befindlichen Stellung arretieren.

10. Vorrichtung nach einem der vorhergehenden Ansprüche 2 bis 7, gekennzeichnet durch an der transportwagenseitigen Seite der Auffahrrampe (16) angeordnete Riegel (21), um die Auffahrrampe (16) in der Anschlußstellung an dem Transportwagen (1) mit diesem in ihre Lage zu verriegeln.

11. Vorrichtung nach Anspruch 9, dadurch gekennzeichnet, daß die Riegel (Arretierung 5) für den Trägerwagen (17) am Transportwagen (2) und die Riegel (21) zum Verbinden der Auffahrrampe (16) mit dem Transportwagen (2) derart miteinander zusammenwirken, daß bei Anschluß der Auffahrrampe (16) an den Transportwagen (2) die Riegel zur Verriegelung des Trägerwagens (7) mit dem Transportwagen entriegelt werden (Fig. 4, Fig. 5).

12. Vorrichtung nach Anspruch 7, dadurch gekennzeichnet, daß die Auffahrrampe (16) verfahrbar ausgebildet ist.

13. Vorrichtung nach Anspruch 2, dadurch gekennzeichnet, daß die haubenartige Textilumhüllung (23) für den Trägerwagen (7) eine dem Griff (14) am Trägerwagen angepaßte Ausformung (24) aufweist.

14. Vorrichtung nach Anspruch 2 und 13, dadurch gekennzeichnet, daß an der Außenseite der haubenartigen Textilumhüllung (23) für den Transportwagen und für den Trägerwagen eine Tasche (25) zur Aufnahme von Transportpapieren od. dgl. vorgesehen ist.

15. Vorrichtung nach Anspruch 2, dadurch gekennzeichnet, daß der Transportwagen mit Gabelstaplerschuhen ausgerüstet ist.

16. Vorrichtung nach Anspruch 2, dadurch gekennzeichnet, daß die rahmenartigen Traggestelle (8, 9, 10) des Trägerwagens (7) matrizenartig die untere Seite der Sterilisationseinheiten (12) umfassen.

## Claims

1. Method for the transport of sterile linen articles to be employed in operating theatres, using so-called sterilization units which are designed as containers for the linen and which have been treated in a sterilizer located at a distance from the operation theatre, characterized in that the sterilization units are stacked in a carrying trolley, the carrying trolley is covered with a hood-like textile wrapping, the carrying trolley so prepared is placed on to a transport trolley, the transport trolley is equipped with a hood-like textile wrapping, and the trolleys thus nested in one another are treated in the sterilizer and subsequently moved to the operating theatre, and after the hood-like textile wrapping of the transport trolley has been removed the carrying trolley equipped with the hood-like textile wrapping, together with the sterilization units arranged on it, is transported into the operating theatre.

2. Device for the sterile transport of sterilization units for operation linen or the like from the sterilizer to the operating theatre by the method according to Claim 1, characterized by
a) a carrying trolley (7) with mountings for the sterilization units (12),
b) a transport trolley (1) for receiving the carrying trolley (7) and having retention means (5) for the carrying trolley (7) arranged on the transport trolley (1),
c) one hood-like wrapping (23), consisting of textile material, each for the carrying trolley (7) and the transport trolley (1).

3. Device according to Claim 2, characterized in that the carrying trolley (7) is formed from one or more horizontal and frame-like supporting stands (8, 9, 10) for the sterilization units (12), the supporting stands (8, 9, 10) being arranged on a central supporting column (11), and the lowest supporting stand (8) being equipped with running rollers (15).

4. Device according to Claims 2 and 3, characterized in that one or more of the running rollers (3, 15) are designed as steering rollers.

5. Device at least according to Claim 3, characterized in that a handle (14) projecting beyond the front horizontal alignment of the carrying trolley (7) is arranged on one of the frame-like supporting stands (10).

6. Device according to Claim 2, characterized in that the transport trolley (1) is composed of a baseplate (2) equipped with running rollers (3) and of a trellis structure (4) attached on top of this and surrounding the transport trolley (1) on three sides.

7. Device according to Claims 2 and 3, characterized by a run-up ramp (16) for connecting the baseplate (2) of the transport trolley (1) to a building floor (Figure 2).

8. Device according to Claim 7, characterized by guide rails (17, 18) for guiding the rollers (15) of the carrying trolley (7) on the run-up ramp (16) (Figure 2).

9. Device according to one of the preceding Claims 2-8, characterized by mutually interacting locking means (6, 26) which are arranged on the carrying trolley (7) and the transport trolley (1) and which, when the carrying trolley (7) is placed on the transport trolley (1), retain the said carrying trolley (7) in its position on the transport trolley (1).

10. Device according to one of the preceding Claims 2 to 7, characterized by locking means (21) arranged on the side of the run-up ramp (16) facing the transport trolley, in order to lock the run-up ramp (16) in place on the transport trolley (1) in the position of connection to the latter.

11. Device according to Claim 9, characterized in that the locking means (retention means 5) for connecting the carrying trolley (7) to the transport trolley (2) and the locking means (21) for connecting the run-up ramp (16) to the transport trolley (2) mutually interact in such a way that, when the run-up ramp (16) is connected to the transport trolley (2), the locking means for locking the carrying trolley (7) to the transport trolley are released (Figure 4, Figure 5).

12. Device according to the Claim 7, characterized in that the run-up ramp (16) is made moveable.

13. Device according to Claim 2, characterized in that the hood-like textile wrapping (23) for the carrying trolley (7) has a shaped-out portion (24) matching the handle (14) on the carrying trolley.

14. Device according to Claims 2 and 13, characterized in that a pouch (25) for receiving transport papers or the like is provided on the outer face of the hood-like textile wrapping (23) for the transport trolley and for the carrying trolley.

15. Device according to Claim 2, characterized in that the transport trolley is equipped with forklift shoes.

16. Device according to Claim 2, characterized in that the frame-like support stands (8, 9, 10) of the carrying trolley (7) surround the underside of the sterilization units (12) in the manner of a matrix.


**Revendications**

1. Procédé pour transport de linges stériles devant être utilisés dans des salles d'opération, mettant en œuvre des unités dites de stérilisation qui sont conçues comme des récipients pour le linge et qui ont traitées dans un stérilisateur éloigné de la salle d'opération, caractérisé en ce que les unités de stérilisation sont empilées dans un chariot porteur, le chariot porteur est recouvert d'une housse textile en forme de capot, le chariot porteur ainsi recouvert est placé sur un chariot de transport, le chariot de transport est muni d'une housse textile en forme de capot et les chariots ainsi emboîtés l'un dans l'autre sont traités dans le stérilisateur et emmenés ensuite dans la salle d'opération, le chariot porteur avec les unités de stérilisation disposées sur celui-ci, muni de la housse textile en forme de capot étant transporté dans la salle d'opération après avoir retiré la housse textile en forme de capot du chariot de transport.

2. Dispositif pour transport stérile d'unités de stérilisation de linge opératoire ou de matériel similaire du stérilisateur à la salle d'opération, selon le procédé conforme à la revendication 1, caractérisé par

a) un chariot porteur (7) avec des supports pour les unités de stérilisation (12),

b) un chariot de transport (1) pour recevoir le chariot porteur (7), muni de butoirs (5) pour le chariot porteur (7) disposé sur le chariot de transport (1),

c) des housses en forme de capot en matière textile (23) respectivement pour le chariot porteur (7) et le chariot de transport (1).

3. Dispositif conforme à la revendication 2 caractérisé en ce que le chariot porteur (7) est formé d'un ou plusieurs châssis porteurs horizontaux et en forme de cadre (8, 9, 10) pour les unités de stérilisation (12), les châssis porteurs (8, 9, 10) étant montés sur une colonne porteuse centrale (11) et le châssis porteur inférieur (8) étant muni de roulettes de déplacement (15).

4. Dispositif conforme aux revendications 2 et 3, caractérisé en ce qu'une ou plusieurs des roulettes de déplacement (3, 15) sont conçues sous la forme de roulettes de direction.

5. Dispositif conforme au moins à la revendication 3, caractérisé en ce que, sur un des châssis porteurs en forme de cadre (10) est fixée une poignée (14) qui déborde de l'alignement horizontal avant du chariot porteur (7).

6. Dispositif conforme à la revendication 2, caractérisé en ce que le chariot de transport (1) est constitué d'un socle (2), muni de roulettes de déplacement (3), et d'un cadre en grilles (4) relié vers le haut sur celui-ci et entourant le chariot de transport (1) sur trois côtés.

7. Dispositif conforme à la revendication 2 et 3, caractérisé par une rampe d'accès (16) pour relier le socle (2) du chariot de transport (1) au sol d'un bâtiment (Fig. 2).

8. Dispositif conforme à la revendication 7, caractérisé par les glissières de guidage (17, 18) pour guider les roulettes (15) du chariot porteur (7) sur la rampe d'accès (16) (Fig. 2).

9. Dispositif conforme à l'une des revendications précédentes 2 à 8, caractérisé par des verrous coopérant ensemble (6, 26), qui sont disposés sur le chariot porteur (7) et sur le chariot de transport (1) et qui, lorsque le chariot porteur (7) est placé sur le chariot de transport (1), l'immobilisent dans sa position sur le chariot de transport (1).

10. Dispositif conforme à l'une des revendications précédentes 2 à 7, caractérisé par des verrous (21) disposés sur le côté chariot de transport de la rampe d'accès (16) pour verrouiller la rampe d'accès (16) avec le chariot de transport (1) lorsqu'elle est en position de liaison avec ce dernier.

11. Dispositif conforme à la revendication 9, caractérisé en ce que les verrous (butoir 5) pour le chariot porteur (7) sur le chariot de transport (2) et les verrous (21) pour relier la rampe d'accès (16) au chariot de transport (2) coopèrent ensemble de façon que, lorsque la rampe d'accès (16) est reliée au chariot de transport (2), les verrous pour verrouiller le chariot porteur (7) avec le chariot de transport sont déverrouillés (Fig. 4, Fig. 5).

12. Dispositif conforme à la revendication 7, caractérisé ce que la rampe d'accès (16) est conçue pour pouvoir se déplacer.

13. Dispositif conforme à la revendication 2, caractérisé en ce que la housse textile en forme de capot (23) pour le chariot porteur (7) comporte une pièce moulée (24) adaptée à la poignée (14) du chariot porteur.

14. Dispositif conforme aux revendications 2 et 13, caractérisé en ce que, sur la partie extérieure des housses textiles en forme de capot (23) pour le chariot de transport et pour le chariot porteur, est prévue une poche (25) pour recevoir des documents de transport ou des documents similaires.

15. Dispositif conforme à la revendication 2, caractérisé en ce que le chariot de transport est équipé de sabots pour une empileuse à fourche.

16. Dispositif conforme à la revendication 2, caractérisé en ce que les châssis porteurs en forme de cadre (8, 9, 10) du chariot porteur (7) entourent la partie inférieure des unités de stérilisation (12) à la manière de matrices.

# Fig. 1

Fig. 2

# Fig. 5

Fig.4

Fig.5